**Europäisches Patentamt**

(19) **European Patent Office**

**Office européen des brevets**

(11) Numéro de publication: **0 143 064 B1**

(12) ## FASCICULE DE BREVET EUROPEEN

(45) Date de publication du fascicule du brevet:
07.01.88

(51) Int. Cl.⁴: **A 61 M 1/30**

(21) Numéro de dépôt: **84420189.7**

(22) Date de dépôt: **08.11.84**

(54) **Rein artificiel à aiguille unique.**

(30) Priorité: **23.11.83 FR 8318892**

(43) Date de publication de la demande:
**29.05.85 Bulletin 85/22**

(45) Mention de la délivrance du brevet:
**07.01.88 Bulletin 88/1**

(84) Etats contractants désignés:
**BE CH DE GB IT LI NL SE**

(56) Documents cités:
**EP - A - 0 071 951**
**DE - A - 2 636 290**
**DE - B - 2 754 810**
**FR - A - 2 513 884**

(73) Titulaire: **HOSPAL INDUSTRIE, 7, Avenue Lionel Terray, F-69330 Meyzieu (FR)**

(72) Inventeur: **Chevallet, Jacques, 8, route de Ternay, F-69360 Serezin du Rhone (FR)**
Inventeur: **Roy, Olivier, 13, avenue Alsace-Lorraine, F-74100 Annemasse (FR)**

(74) Mandataire: **Gauckler, Jacques et al, Service Brevets HOSPAL HOSPAL C.O.T. B.P. 21, F-69881 Meyzieu Cedex (FR)**

ACTORUM AG

## Description

La présente invention concerne un perfectionnement apporté à un rein artificiel comportant un circuit extracorporel de sang relié au réseau artério-veineux d'un patient par un accès unique tel que aiguille ou catheter, en forme de Y ou de T. Elle concerne plus spécialement un rein artificiel dont le circuit extracorporel de sang comporte un dispositif de traitement de sang et notamment de purification du sang par passage du sang sur une membrane semi-perméable, tel qu'un hémodialyseur et/ou un hémofiltre, lequel est encadré par deux pompes de circulation du sang, la pompe artérielle étant disposée à l'amont du dispositif de traitement de sang et la pompe veineuse étant disposée à l'aval de ce dispositif.

Des reins artificiels du type à aiguille unique sont déjà connus. Ainsi, la demande EP 71 557 décrit un tel type de rein artificiel dont le circuit extracorporel de sang comporte en outre un dispositif (34) susceptible d'amortir les pulsations du sang. De tels reins artificiels présentent l'avantage par rapport aux reins traditionnels reliés aux patients par deux aiguilles indépendantes de ne nécessiter à chaque traitement qu'un accès unique au réseau artério-veineux du patient, ce qui est considérablement moins traumatisant.

Les reins artificiels à aiguille unique à deux pompes, artérielle et veineuse, ont une efficacité comparable à celle d'un rein artificiel classique à deux aiguilles. En outre ils permettent une meilleure maîtrise de la pression moyenne du sang entre les deux pompes, ce qui permet un meilleur contrôle du prélèvement de l'ultrafiltrat.

Mais un problème apparaît lorsque l'on doit retirer au patient des quantités relativement importantes d'ultrafiltrat qu'il faut généralement aussitôt compenser par la perfusion de volumes sensiblement équivalents de liquide physiologique et/ou de solutions stériles, à la perte de poids près, désirée pour le patient.

Un exemple de perfusion de solution saline dans un circuit sanguin est décrit dans la demande de brevet FR 2 513 884.

Cependant si l'on effectue ces perfusions selon un débit continu, constant ou variable, du fait du retour pulsé du sang au patient, on risque de lui renvoyer un mélange encore non homogénéisé sang-solution perfusée, qui peut dans certains cas provoquer chez lui des troubles physiologiques.

Ces inconvénients peuvent encore être accentués par un choix défavorable de l'emplacement du point de perfusion sur le circuit extracorporel. D'une part si le point de perfusion est situé en amont de l'appareil de traitement de sang, une partie du liquide perfusé pourra s'éliminer aussitôt par ultrafiltration n'atteignant jamais le patient. D'autre part, si l'on effectue la perfusion en aval de l'appareil de traitement de sang, mais en un point éloigné de la pompe veineuse, il y a risque d'envoyer au patient un mélange sang/solution perfusée très hétérogène qui peut être aggravé par un risque de recirculation d'une partie de la solution perfusée dans le circuit à aiguille unique, via l'aiguille unique et la pompe artérielle.

De plus, dans les reins artificiels munis de dispositifs de mesure et de contrôle de la pression veineuse, la perfusion de solutions peut créer des variations de pression susceptibles de déclencher des alarmes intempestives.

Un objet de la présente invention est de proposer un rein artificiel poyvalent qui permette de bénéficier des avantages des circuits à aiguille unique à double pompe et en outre de pratiquer des traitements d'épuration du sang par hémodialyse et/ou par ultrafiltration-réinjection avec des taux élevés.

Un autre objet de la présente invention est un rein artificiel qui permette des traitements d'ultrafiltration-réinjection offrant d'excellentes garanties de régularité, de fiabilité et de sécurité sur un plan physiologique.

Un autre objet de la présente invention est un rein artificiel dont le fonctionnement soit simple, économique, automatique, fiable et sûr.

Il a maintenant été trouvé, et ceci fait l'objet de la présente invention à la réalisation de laquelle ont collaboré Messieurs Jacques Chevallet et Olivier Roy, un rein artificiel comprenant un circuit extracorporel de sang du type à aiguille unique, ledit circuit traversant un appareil de traitement de sang à membrane sélectivement perméable, tel qu'un hémodialyseur ou un hémofiltre, relié le cas échéant à au moins un dispositif susceptible d'amortir les pulsations du sang et encadré par deux pompes de circulation du sang qui fonctionnent de manière séquentielle, une pompe artérielle étant disposée à l'amont et une pompe veineuse à l'aval dudit appareil de traitement de sang, caractérisé en ce qu'il comporte des moyens de perfusion d'au moins une solution dans ledit circuit extracorporel de sang, lesdits moyens de perfusion comprenant au moins une pompe de perfusion dont les moyens d'entraînement séquentiels de l'une desdites pompes de circulation du sang.

La compréhension de la présente invention sera facilitée par les figures ci-jointes qui illustrent, à titre d'exemples, schématiquement et sans échelle déterminée, divers modes de réalisation du rein artificiel selon l'invention.

La figure 1 représente un premier mode de réalisation du rein artificiel selon l'invention.

La figure 2 représente un deuxième mode de réalisation du rein artificiel selon l'invention.

La figure 3 représente des variantes de réalisation du rein artificiel selon l'invention.

Pour plus de commodité, les éléments homologues des reins artificiels représentés dans les différentes figures sont désignés par les mêmes numéros.

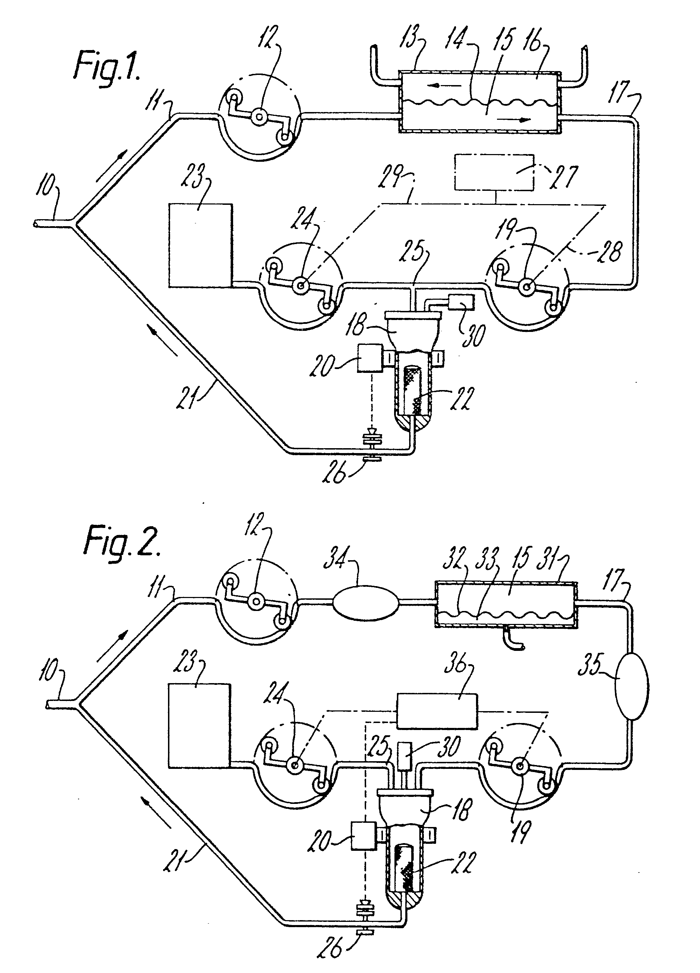

En se référant d'abord à la figure 1, on voit que le sang est prélevé au patient au moyen d'un accès unique de tout type connu en soi, tel qu'une aiguille ou un catheter, en forme de Y ou de T. Le sang parcourt un circuit extracorporel composé essentiellement d'un appareil de traitement, équipé d'une membrane sélectivement perméable, tel que hémodialyseur et/ou hémofiltre, de pompes de circulation, d'accessoires divers dont seuls les principaux ont été représentés et de lignes de liaison entre ces divers dispositifs.

Ainsi dans la ligne (11) composée généralement d'un tube flexible en chlorure de polyvinyle, le sang est conduit par une pompe (12), par exemple du type péristaltique, de l'aiguille (10) à un hémodialyseur (13). Cette première pompe (12), alimentant l'hémodialyseur et disposée à l'amont de celui-ci, véhicule du sang qui se trouve à l'origine à la pression artérielle du patient et elle est de ce fait appelée «pompe artérielle». L'hémodialyseur (13), de tout type connu en soi, comprend essentiellement une membrane (14) permettant la dialyse et l'ultrafiltration du sang, elle le sépare en deux compartiments (15 et 16), le premier parcouru par le sang et le second par le liquide de dialyse qui peut entraîner en outre l'ultrafiltrat.

Une ligne (17), de type semblable à la ligne (11), conduit généralement le sang de l'hémodialyseur (13) à un dispositif débulleur (18) de tout type connu en soi, au moyen d'une seconde pompe (19), disposée à l'aval de l'hémodialyseur, refoulant le sang traité chez le patient à la pression veineuse du patient; elle est de ce fait appelée «pompe veineuse» et elle est généralement d'une type semblable, voire identique à la pompe artérielle (12).

Le débulleur a pour rôle essentiel de retenir toute bulle d'air qui risquerait d'être entraînée par le sang dans le circuit extracorporel vers le patient. La présence de bulles d'air ou de mousse constituée par l'agglomération de bulles d'air très fines, est détectée par le dispositif (20) qui commande prioritairement la fermeture automatique d'une pince occlusive ou «clamp» (26) de tout type connu en soi disposé sur la ligne (21) reliant directement le débulleur (18) au dispositif d'aiguille unique (10). Avantageusement le fonctionnement du «clamp» (26) est en outre synchronisé avec le fonctionnement de la pompe veineuse (19), c'est-à-dire qu'il est ouvert lorsque la pompe (19) tourne. Préférentiellement un filtre (22), disposé au fond du débulleur (18), retient les impuretés, les poussières, ou les éléments hémolysés, avant le retour su sang au patient. En outre, on dispose généralement un dispositif (30) pour mesurer la pression du sang en un point quelconque du circuit de retour entre la pompe (19) et l'aiguille (10), de préférence entre la pompe (19) et le «clamp» (26), par exemple avantageusement sur le débulleur (18).

Généralement le «clamp (26) est disposé en amont de l'aiguille unique (10) d'une part et en aval de tout autre organe sur le circuit de retour du sang au patient d'autre part. Ceci permet de limiter considérablement les risques de fuite de sang ou d'entrée d'air intempestive, par exemple à un simple raccord de tuyauterie qui serait défectueux. Naturellement on peut placer le dispositif de mesure de pression (30) sans inconvénient à l'aval du clamp (26) dans la mesure où il est disposé autour du conduit (21) et n'est en contact qu'avec la paroi extérieure de ce conduit, sans nécessiter de raccord spécifique.

Le fonctionnement de ce type de rein artificiel est séquentiel, le sang parcourant l'aiguille unique (10), alternativement du patient vers l'hémodialyseur (13) puis, de celui-ci vers le patient. Les pompes à sang (12) et (19) ont donc généralement un fonctionnement discontinu et décalé dans le temps. La régulation s'effectue soit par programmation des durées de fonctionnement respectives, soit de préférence, en fonction de la pression du sang entre les deux pompes, pour éviter tout écart excessif de pression. Généralement une pression anormale, détectée par le dispositif (30) entraîne l'arrêt de la pompe veineuse (19).

Tous les éléments du rein artificiel décrits jusqu'ici sont bien connus et ne seront donc pas décrits ici plus en détails.

Selon la présente invention on a trouvé des moyens permettant de retirer à un patient bénéficiant d'un traitement par rein artificiel à aiguille unique à double pompe de circulation, des quantités d'ultrafiltrat relativement élevées, par exemple de l'ordre de 3 à 4 litres par séance, ou même davantage et de les compenser, à la perte de poids près désirée pour le patient, par des perfusions de volumes sensiblement équivalents de solutions physiologiques, généralement isotoniques au sang.

Ces moyens de perfusion sont constitués par un réservoir (23) de tout type connu en soi et de la capacité désirée, relié à une pompe de perfusion (24), par exemple une pompe péristaltique occlusive; celle-ci est reliée au circuit extracorporel de sang par un conduit de perfusion, en un point de perfusion tel que (25). Les moyens d'entraînement de la pompe de perfusion sont synchronisés aux moyens d'entraînement de l'une des deux pompes de circulation du sang, généralement celle qui est la plus proche du point de perfusion. On a observé qu'ainsi il est possible de faire arriver simultanément au point de perfusion le sang et le liquide de perfusion, dans un rapport de débits prédéterminé et avantageusement réglable. On a observé également qu'à l'aval du point de perfusion le sang et le liquide de perfusion se mélangent de manière sensiblement homogène dans des proportions à peu près constantes et que ce mélange peut être injecté au patient sans causer de troubles physiologiques.

Le dispositif (27) représente schématiquement un organe de commande de tout type connu en soi, par exemple équipé d'un programmateur, qui agit, par exemple électriquement ou électroniquement, via les lignes (28) et (29), respectivement sur les moteurs des pompes (19) et (24).

Selon l'invention, on a trouvé avantageux de placer le point d'injection dans le circuit extracorporel de sang à proximité de l'orifice amont ou aval de la pompe de circulation dont les moyens d'entraînement sont synchronisés aux moyens d'entraînement de la pompe de perfusion. Par placé à proximité, on entend essentiellement qu'il n'y a pas d'organe important tel qu'un débulleur, un dispositif amortisseur de pulsations ou une vanne, susceptible de modifier le dosage désiré du liquide de perfusion par rapport au sang, interposé entre la pompe et le point d'injection, la distance entre ces deux éléments étant secondaire, car généralement faible. C'est ainsi que sur la figure 1, le point d'injection (25) apparaît situé à proximité et à l'aval de la pompe veineuse (19).

La figure 2 montre un second mode de réalisation du rein artificiel selon l'invention dont certaines dispositions peuvent être mises en œuvre séparément ou en diverses combinaisons avec les autres modes de réalisation représentés.

Ainsi la pompe de perfusion (24) et la pompe veineuse (19) peuvent être entraînées par un moteur commun (36). Avantageusement elles peuvent ainsi être entraînées avec un synchronisme rigoureux aux mêmes moments et aux mêmes vitesses ou dans un même rapport de vitesses. Ce rapport de vitesses peut toutefois être réglable à l'aide d'un variateur de vitesses de tout type connu afin de permettre d'ajuster les rapports du débit de la solution perfusée par rapport au débit sanguin.

De préférence la pompe de perfusion (24) et la pompe veineuse (19) sont de type péristaltique. Avantageusement les tubes péristaltiques des pompes (19) et (24) sont montés en parallèle sur un même rotor. Les diamètres d'enroulement des tubes péristaltiques sur le rotor, ainsi que les diamètres internes et/ou externes des tubes péristaltiques sont préalablement déterminés en fonction du rapport des débits moyens ou des capacités de débit souhaités, dans le cas de perfusions avec rapport de vitesses fixe.

Un dispositif de fermeture à pince non représenté, disposé par exemple entre le réservoir (23) et la pompe de perfusion (24) peut, le cas échéant, permettre de limiter — au moins temporairement — le débit de liquide perfusé dans le sang, sans que l'on ait à modifier le mouvement de la pompe (24).

En outre il est avantageux de prévoir dans le réservoir (23) un dispositif détecteur de flacon vide d'un type connu en soi, non représenté, capable de commander si nécessaire l'arrêt de la pompe de perfusion (24) pour éviter l'introduction d'air dans le sang.

D'autre part, les liquides débités par chacune des pompes (19 et 24) sont envoyés directement en parallèle au sommet du débulleur (18) dans lequel s'effectue leur mélange dans les proportions désirées. On peut donc encore considérer figure 2 que le point de perfusion (25) est situé à proximité de l'orifice aval de la pompe veineuse (19), synchronisée à la pompe de perfusion (24).

Par ailleurs la figure 2 représente comme appareil de traitement de sang un hémofiltre (31) muni d'une membrane d'ultrafiltration (32) séparant l'appareil en deux compartiments (15 et 33), parcourus respectivement par le sang et par l'ultrafiltrat. Cet hémofiltre est de tout type connu. Lorsqu'il est constitué par exemple par un faisceau de fibres creuses sensiblement parallèles parcourues intérieurement par le sang, il offre au sang un volume relativement invariable conduisant, en fonction du mouvement alternatif des pompes à sang, à de fortes variations de pression du sang. On a trouvé alors avantageux de disposer sur le circuit de sang un ou deux dispositifs amortisseurs de pulsations (34 et 35) d'un type connu en soi, par exemple à l'amont et à l'aval de l'appareil de traitement de sang. On obtient ainsi un écoulement moins hémolysant et plus régulier, favorable à un mélange homogène avec des quantités importantes de liquide de perfusion.

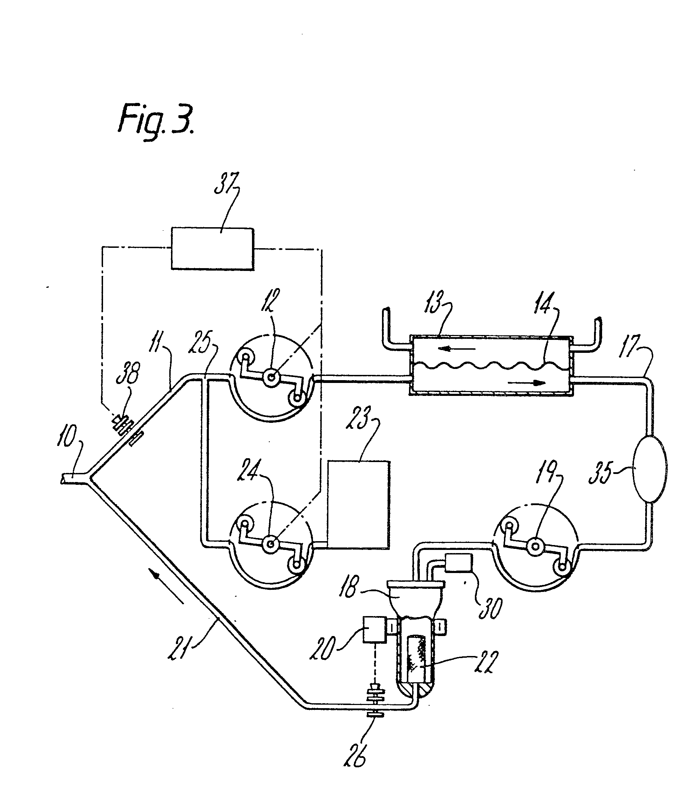

La figure 3 montre une autre combinaison de moyens. Ainsi une pince occlusive ou «clamp» (38) est disposée sur la ligne (11), à l'amont de la pompe artérielle (12). Le fonctionnement de ce «clamp» est dit automatique, car synchronisé à celui de la pompe (12) par l'intermédiaire d'un boitier de commande électrique (37), de façon à éviter une aspiration parasite de sang veineux depuis la ligne (21).

Selon l'invention, le point de perfusion est placé à l'amont de l'une des pompes de circulation, par exemple à l'amont de la pompe artérielle (12) et à proximité immédiate de l'aspiration de cette pompe. En outre les moyens d'entraînement de la pompe de perfusion (24) sont synchronisés aux moyens d'entraînement de la pompe artérielle (12), par tous moyens connus, par exemple par l'intermédiaire du boitier de commande (37). Ainsi le sang et le liquide de perfusion se déplacent-ils simultanément et se mélangent-ils ensuite sur le reste du circuit extracorporel avant le retour au patient. Cependant cette disposition peut présenter l'inconvénient qu'une partie du liquide de perfusion quitte le circuit extracorporel de sang peu après y avoir été introduite, en traversant la membrane (14), aussi constitue-t-elle une variante non préférentielle.

Le fonctionnement du rein artificiel selon la figure 3 est séquentiel. Dans un premier temps, la pompe veineuse (19) étant arrêtée, le programmateur du boitier (37) déclenche l'ouverture du clamp (38) puis la rotation simultanée de la pompe artérielle (12) et de la pompe de perfusion (24). Lorsque la pression aval mesurée par une sonde (non représentée) disposée par exemple sur le dispositif amortisseur de pulsations (35) atteint une valeur maximum prédéterminée, le programmateur (37) arrête les pompes (12) et (24) et ferme le clamp (38), tandis que dans un deuxième temps, la pompe veineuse (19) est entraînée à son tour en rotation jusqu'à ce que la pression amont mesurée en (35) atteigne une valeur minimum prédéterminée.

D'une manière générale on a trouvé avantageux d'entraîner la pompe de perfusion (24) de façon discontinue, périodique, avec une période calée sur la période de fonctionnement de l'une des deux pompes de circulation, artérielle ou veineuse et de préférence de la pompe veineuse. Ainsi les moyens d'entraînement de la pompe de perfusion d'une solution sont avantageusement synchronisés aux moyens d'entraînement de l'une des deux pompes de circulation, notamment de la pompe veineuse.

Si désiré, et bien que ça ne soit pas préférentiel, on peut faire fonctionner la pompe de perfusion (24) pendant une période de durée différente de celle de la pompe veineuse (19). Ainsi, lorsque par exemple les volumes à perfuser sont relativement faibles, on peut n'entraîner la pompe de perfusion (24) que pendant une fraction du temps de fonctionnement de la pompe veineuse (19) à chaque période.

Selon l'invention, le mouvement de la pompe de perfusion (24) est directement synchronisé avec celui de l'une au moins des deux pompes de circulation (12) ou (19). Il en résulte qu'il est aussi synchronisé avec la position des clamps amont ou aval, tels que (38) ou (26), généralement indirectement. En effet on remarque par exemple que la pompe de perfusion (24) ne fonctionne que lorsque le clamp correspondant (38) ou (26), proche du point de perfusion (25) est ouvert.

L'invention permet de perfuser tous types de liquides physiologiques ou de solutions stériles, préalablement dosées en fonction des besoins du patient. On peut ainsi corriger des besoins en sels minéraux, en protéines, etc...

On peut aussi injecter plusieurs solutions successivement ou simultanément, par exemple en parallèle, dans des proportions déterminées ou indépendantes, à l'aide de plusieurs réservoirs (23) et/ou de pompes (24). Naturellement on peut en outre injecter à l'amont du circuit extracorporel de sang et indépendamment, de l'héparine, comme il est déjà bien connu de le faire.

La commande séquentielle des moyens de perfusion peuvent naturellement faire l'objet de nombreuses variantes de réalisation à la portée du technicien, sans sortir du cadre de la présente invention.

## Revendications

1. Rein artificiel comprenant un circuit extracorporel de sang du type à aiguille unique, ledit circuit traversant un appareil de traitement de sang à membrane sélectivement perméable, tel qu'un hémodialyseur (13) ou un hémofiltre (31), relié le cas échéant à au moins un dispositif (34, 35) susceptible d'amortir les pulsations du sang et encadré par deux pompes (12, 19) de circulation du sang, qui fonctionnent de manière séquentielle, une pompe artérielle (12) étant disposée à l'amont et une pompe veineuse (19) à l'aval dudit appareil de traitement de sang, caractérisé en ce qu'il comporte des moyens de perfusion (23, 24, 25) d'au moins une solution dans ledit circuit extracorporel de sang, lesdits moyens de perfusion comprenant au moins une pompe de perfusion (24) dont les moyens d'entraînement sont synchronisés aux moyens d'entraînement séquentiels de l'une desdites pompes de circulation de sang (12, 19).

2. Rein artificiel selon la revendication 1, caractérisé en ce que lesdits moyens de perfusion sont reliés audit circuit extracorporel de sang en un point (25) situé à proximité de l'orifice amont ou aval de ladite pompe de circulation (12, 19) dont les moyens d'entraînement sont synchronisés aux moyens d'entraînement de ladite pompe de perfusion (24).

3. Rein artificiel selon l'une des revendications 1 ou 2, caractérisé en ce que les moyens d'entraînement de la pompe de perfusion (24) d'une solution sont synchronisés aux moyens d'entraînement de la pompe veineuse (19).

4. Rein artificiel selon l'une quelconque des revendications précédentes, caractérisé en ce que les moyens d'entraînement de ladite pompe de perfusion (24) sont synchronisés aux moyens d'entraînement séquentiels de l'une desdites pompes de circulation de sang (12, 19) dans un rapport de vitesses réglable.

5. Rein artificiel selon l'une quelconque des revendications précédentes, caractérisé en ce que les moyens d'entraînement de la pompe de perfusion (24) d'une solution et de la pompe de circulation (19) qui lui est synchronisée sont commandés par un organe moteur commun (36).

6. Rein artificiel selon l'une quelconque des revendications précédentes, caractérisé en ce que lesdites pompes de perfusion d'une solution (24) et de circulation du sang (12, 19) qui lui est synchronisée sont de type péristaltique.

7. Rein artificiel selon la revendication 6, caractérisé en ce que lesdites pompes synchronisées (24, 19) ont un rotor commun actionnant simultanément deux tubes péristaltiques montés en parallèle sur ledit rotor.

8. Rein artificiel selon l'une quelconque des revendications précédentes, caractérisé en ce qu'un dispositif de femeture (26) du circuit extracorporel de sang est disposé sur le circuit de retour (21) du sang au patient en amont de l'aiguille unique (10) d'une part et en aval de tout autre organe d'autre part.

9. Rein artificiel selon la revendication 8, caractérisé en ce que la commande dudit dispositif de fermeture (26) est synchronisé aux moyens d'entraînement (36) de la pompe de perfusion (24) et/ou de la pompe veineuse (19).

## Patentansprüche

1. Künstliche Niere mit einem ausserhalb des Körpers gelegenen Blutkreislauf, von der Bauart mit einziger Nadel, wobei der Kreislauf ein Blutbehandlungsgerät, z. B. einen Hämodialysator (13) oder einen Hämofilter (31), mit selektiv durchlässiger Membran durchquert, das gegebenenfalls mit wenigstens einer Vorrichtung (34, 35) verbunden ist, die das Pulsieren des Bluts dämpfen kann und von zwei Pumpen (12, 19) für die Blutzirkulation begrenzt ist, die aufeinanderfolgend arbeiten, wobei eine arterielle Pumpe (12) stromauf und eine venöse Pumpe (19) stromab vom Blutbehandlungsgerät angeordnet ist, dadurch gekennzeichnet, dass sie Mittel (23, 24, 25) zur Perfusion von wenigstens einer Lösung in den ausserhalb des Körpers gelegenen Blutkreislauf aufweist, wobei die Perfusionsmittel wenigstens eine Perfusionspumpe (24) umfassen, deren Antriebsmittel mit den Mitteln für den aufeinanderfolgenden Antrieb einer der Blutzirkulationspumpen (12) synchronisiert sind.

2. Künstliche Niere nach Anspruch 1, dadurch gekennzeichnet, dass die Perfusionsmittel mit dem ausserhalb des Körpers gelegenen Kreislauf an einem Punkt (25) verbunden sind, der sich in der Nähe der stromauf oder stromab gelegenen Öffnung der Zirkulationspumpe (12, 19) befindet, deren Antriebsmittel mit den Antriebsmitteln der Perfusionspumpe (24) synchronisiert sind.

3. Künstliche Niere nach einem der Ansprüche 1 oder 2, dadurch gekennzeichnet, dass die Antriebsmittel der Perfusionspumpe (24) für eine Lösung mit den Antriebsmitteln der venösen Pumpe (19) synchronisiert sind.

4. Künstliche Niere nach einem der vorhergehenden Ansprüche, dadurch gekennzeichnet, dass die Antriebsmittel der Perfusionspumpe (24) mit den aufeinanderfolgenden Antriebsmitteln einer der Blutzirkulationspumpen (12, 19) in einem regelbaren Geschwindigkeitsverhältnis synchronisiert sind.

5. Künstliche Niere nach einem der vorhergehenden Ansprüche, dadurch gekennzeichnet, dass die Antriebsmittel der Perfusionspumpe (24) für eine Lösung und der mit ihr synchronisierten Zirkulationspumpe (19) von einem gemeinsamen Antriebsorgan (36) gesteuert werden.

6. Künstliche Niere nach einem der vorhergehenden Ansprüche, dadurch gekennzeichnet, dass die Perfusionspumpen (24) für eine Lösung und die hiermit synchronisierte Blutzirkulationspumpe (12, 19) von peristaltischer Bauart sind.

7. Künstliche Niere nach Anspruch 6, dadurch gekennzeichnet, dass die synchronisierten Pumpen (24, 19) einen gemeinsamen Rotor haben, der gleichzeitig zwei peristaltische Rohre betätigt, die am Rotor parallel angebracht sind.

8. Künstliche Niere nach einem der vorhergehenden Ansprüche, dadurch gekennzeichnet, dass eine Schliessvorrichtung (26) für den ausserhalb des Körpers gelegenen Blutkreislauf am Kreislauf (21) für den Rücklauf des Bluts zum Patienten stromauf der einzigen Nadel (10) einerseits und stromab von jedem anderen Organ angeordnet ist.

9. Künstliche Niere nach Anspruch 8, dadurch gekennzeichnet, dass die Steuerung der Schliessvorrichtung mit den Antriebsmitteln (36) der Perfusionspumpe (24) und/oder der venösen Pumpe (19) synchronisiert ist.

**Claims**

1. Artificial kidney comprising a blood circuit of the single-needle type, external to the body, the said circuit passing through a blood treatment apparatus with a selectively permeable membrane, such as a blood dialyser (13) or a blood filter (31), connected, where appropriate, to at least one device (34, 35), capable of absorbing the blood pulsations and enclosed between two blood circulation pumps (12, 19) which operate sequentially, an arterial pump (12) being arranged upstream and a venous pump (19) downstream of the said blood treatment apparatus, characterized in that it comprises means for perfusion (23, 24, 25) of at least one solution into the said blood circuit external to the body, the said means of perfusion comprising at least one perfusion pump (24) whose driving means are synchronized with the sequential means for driving one of the said blood circulation pumps (12, 19).

2. Artificial kidney according to Claim 1, characterized in that the said means of perfusion are connected to the said blood circuit external to the body at a point (25) situated close to the upstream or downstream orifice of the said circulation pump (12, 19) whose driving means are synchronized with the means for driving the said perfusion pump (24).

3. Artificial kidney according to either of Claims 1 and 2, characterized in that the means for driving the pump for perfusion (24) of a solution are synchronized with the means for driving the venous pump (19).

4. Artificial kidney according to any one of the preceding claims, characterized in that the means for driving the said perfusion pump (24) are synchronized with the sequential means for driving one of the said blood circulation pumps (12, 19) in a speed relationship which is adjustable.

5. Artificial kidney according to any one of the preceding claims, characterized in that the means for driving the pump for perfusion (24) of a solution and of the circulation pump (19) which is synchronized with it are controlled by a common driving device (36).

6. Artificial kidney according to any one of the preceding claims, characterized in that the said pumps for perfusion of a solution (24) and for blood circulation (12, 19) which is synchronized with it are of a peristaltic type.

7. Artificial kidney according to Claim 6, characterized in that the said synchronized pumps (24, 19) have a common rotor simultaneously actuating two peristaltic tubes mounted in parallel on the said rotor.

8. Artificial kidney according to any one of the preceding claims, characterized in that a device for closing (26) the blood circuit external to the body is arranged in the circuit for returning (21) the blood to the patient upstream of the single needle (10), on the one hand, and downstream of any other member, on the other hand.

9. Artificial kidney according to Claim 8, characterized in that the control of the said closure device (26) is synchronized with the means for driving (36) the perfusion pump (24) and/or the venous pump (19).

Fig.1.

Fig.2.

# Fig. 3.